# FASCICULE DE BREVET EUROPEEN

(11) **EP 0 974 055 B1**
(45) Date de publication et mention de la délivrance du brevet: **26.03.2003**
(21) Numéro de dépôt: 98917302.6
(22) Date de dépôt: 03.04.1998
(51) Int. Cl.: G01N 33/569, C12Q 1/06, C12Q 1/70

(54) **METHODE DE TITRAGE D'UNE COMPOSITION VIRALE COMPLEXE**
TITRIERVERFAHREN FÜR KOMPLEXE VIRUSZUSAMMENSETZUNG
TITRATION METHOD FOR A COMPLEX VIRAL COMPOSITION

(30) Priorité: 04.04.1997 FR 9704371
(43) Date de publication de la demande: 26.01.2000
(73) Titulaire: Aventis Pasteur, 69367 Lyon Cedex 07 (FR)
(72) Inventeur: GERDIL, Catherine, F-69130 Ecully (FR); SALUZZO, Jean-François, F-69005 Lyon (FR)
(74) Mandataire: Kerneis, Daniéle
(86) Numéro de dépôt international: FR9800678
(87) Numéro de publication internationale: WO98045709

(56) Documents cités:
- EP-A- 0 279 563
- FR-A- 2 511 702
- FR-A- 2 694 022
- PATENT ABSTRACTS OF JAPAN vol. 018, no. 596 (P-1825), 14 novembre 1994 & JP 06 222062 A (TEIJIN LTD), 12 août 1994,
- SIMANTINI ET AL: "Epiotope mapping of dengue 1 virus E glycoprotein using monoclonal antibodies" ARCH.VIROLOGY, vol. 140, no. 7, 1995, pages 1257-1273, XP002050424
- OERVELL ET AL: "The effects of monoclonal antibodies against the hemagglutinin-neuraminidase and fusion protein on the release of Sendai virus from infected cells" ARCH.VIROLOGY, vol. 86, no. (1-2), 1985, pages 1-15, XP002050425

## Description

L'invention concerne le domaine des compositions virales; plus particulièrement l'invention est relative à une méthode, pour déterminer, dans une composition contenant différentes espèces ou types de virus vivants, la quantité de virus de chacune des espèces, ou plus particulièrement de chacun des sérotypes d'une espèce donnée de virus.

On connaît dans l'art antérieur, des méthodes pour doser dans une composition virale, la quantité de chaque espèce ou de chaque type de virus. Les méthodes habituellement utilisées consistent à neutraliser, au moyen d'anticorps polyclonaux les virus que l'on ne souhaite pas doser, puis à déterminer la quantité du virus restant. C'est notamment ce qui se produit lorsqu'on veut déterminer dans une composition vaccinale contre la polio, la quantité de virus vivants atténués présente pour chacun des types I, II ou III. Cependant, il n'est pas toujours possible de mettre en oeuvre de telles méthodes car il est parfois difficile, voire impossible, de neutraliser certains des virus présents dans la composition sans interférer avec le virus que l'on veut doser, notamment lorsqu'il s'agit de titrer une composition vaccinale comprenant plusieurs sérotypes d'un virus particulier, tel que le virus de la Dengue par exemple. Il n'y a en effet pas d'anticorps polyclonaux spécifiques des types ; quant aux anticorps monoclonaux capables de reconnaître spécifiquement un type donné, ils sont souvent non suffisamment neutralisants ; parfois même, on ne connaît pas d'anticorps monoclonal neutralisant. Or, dans certains cas, et notamment dans l'industrie des vaccins, il est nécessaire de produire des compositions virales comprenant différentes espèces ou différents sérotypes d'une espèce de virus dans un proportion parfaitement définie. De plus, les exigences pharmaceutiques imposent de pouvoir ensuite contrôler quantitativement, de façon fiable, la composition des produits fabriqués.

Il est donc souhaitable de pouvoir disposer de méthodes permettant de titrer, sans la modifier, une composition virale complexe.

A cette fin, la présente invention a pour objet une méthode pour déterminer, dans une composition contenant différentes espèces ou types de virus vivants, la quantité de virus de chacun des types ou espèces de virus, caractérisée en ce qu'elle comprend les étapes suivantes :
propagation des virus de chaque type ou espèce sur des cellules VERO
dosage de chaque type ou espèce de virus au moyen d'un anticorps monoclonal spécifique.

La méthode selon l'invention s'applique à une composition comprenant plusieurs espèces différentes de virus, et/ou plusieurs sérotypes à l'intérieur d'une même espèce. Il peut notamment s'agir des virus responsables de la poliomyélite, de la rubéole, des oreillons, de la rougeole, de la Dengue, ou encore des rotavirus. Les compositions comprenant ces virus peuvent être des compositions vaccinales, dans lesquelles les virus bien qu'ayant une virulence atténuée, sont maintenus vivants. Il peut ainsi s'agir par exemple d'une composition vaccinale comprenant les trois sérotypes du virus de la polio ou d'une composition comprenant les quatre sérotypes du virus de la Dengue. La méthode selon l'invention est particulièrement intéressante lorsqu'il s'agit de virus très proches antigéniquement et dont la neutralisation d'un sérotype entraîne, par réaction croisée, la neutralisation des autres sérotypes.

Selon l'invention, on procède à la propagation des virus présents dans la composition à tester, à différentes dilutions, sur des cellules Vero.

Les cellules sont disposées dans des cupules de plaques adaptées à la culture de cellules puis inoculées avec des suspensions virales.

Le milieu de culture utilisé pour la propagation virale est un milieu classique adapté aux cellules VERO utilisées et au virus à titrer. Après un temps d'incubation qui varie selon le virus (par exemple une semaine pour le virus de la Dengue), à la température optimale de croissance du virus sous atmosphère de CO₂, les surnageants de cultures cellulaires sont éliminés ; les cellules sont ensuite fixées, par exemple au moyen d'acétone refroidi.

Pour chacune des dilutions réalisées, on détermine ensuite la quantité de virus présents grâce à un anticorps monoclonal spécifique de l'espèce ou du sérotype selon le titrage réalisé. La réaction est révélée par un anticorps anti-espèce marqué à la fluorescéine ou au moyen d'un substrat adapté au test ELISA. Le titre viral est déterminé par la méthode Spearman et Karber et exprimé en dose infectant 50 % des cultures cellulaires (CCID₅₀).

On procède de la même façon, en parallèle, avec chaque anticorps monoclonal spécifique de l'espèce ou du type de virus que l'on souhaite titrer dans la composition virale.

On peut ainsi, grâce à cette méthode, titrer chaque sérotype présent dans la composition virale, et ceci de façon surprenante, sans que l'un des sérotypes soit prédominant sur les autres.

### Exemple

Titrage de quatre compositions vaccinales monovalentes comprenant chacune un sérotype du virus de la Dengue, et d'une composition préparée extemporanément par mélange en quantité égale des quatre compositions monovalentes testées.

On effectue le titrage sur des micro-plaques 96 puits de la manière suivante :
- on réalise des dilutions successives de chacune des compositions, grâce à du milieu de culture MEM, comprenant 5 % de Sérum de Veau Foetal et 2 g/l de bicarbonate de sodium,
- on inocule les suspensions virales ainsi obtenues à des cellules Vero (Réf. ATCC :
   CCL81VERO) en couches établies de 1 jour, à raison de dix cupules par dilution.
   Chaque valence des suspensions monovalentes et de la composition tétravalente est titrée sur au moins une plaque 96 puits.
- on incube ensuite une semaine à 36° C sous 5 % de CO₂,
- on élimine les surnageants de culture cellulaire et on fixe les cellules sur les plaques à l'acétone à - 20° C,
- on détecte ensuite la présence de virus grâce à un anticorps monoclonal spécifique du sérotype présent dans la composition vaccinale. Les anticorps utilisés sont issus d'hybridomes fournis par le CDC (Center of Disease Control, Atlanta, USA).
   Le sérotype international 1 est marqué par l'anticorps issu de l'hybridome D2 - 1F1 - 3
   Le sérotype international 2 est marqué par l'anticorps issu de l'hybridome 3H5 - 1 - 12
   Le sérotype international 3 est marqué par l'anticorps issu de l'hybridome 5D4 - 11 - 24
   Le sérotype international 4 est marqué par l'anticorps issu de l'hybridome 1H10 - 6 - 7
- on révèle la réaction grâce à un anticorps anti-IgG de souris marqué à la fluorescéine.

La lecture est réalisée au microscope à fluorescence. On compte pour chaque dilution le nombre de cupules présentant au moins un foyer de cellules infectées (fluorescentes).
Le titre du produit correspond à la dilution induisant une atteinte de 50 % des nappes cellulaires (soit 50 % des cupules) et est calculé par la méthode de Spearman et Karber. Il est exprimé en log₁₀ de CCID₅₀.

Chacun des dosages étant effectué deux fois, on obtient le tableau des résultats suivants :

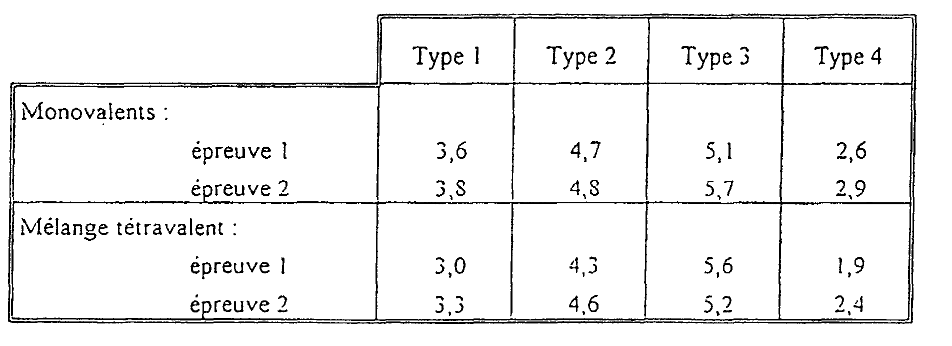

On remarque que les résultats obtenus sont conformes aux résultats attendus ; la différence de titre observée dans le mélange tétravalent pour chaque type de virus varie environ de 0,5 log₁₀CCID₅₀, ce qui correspond à la dilution au 1/4 réalisée pour chaque sérotype lors de la réalisation du mélange.

Ainsi, selon l'invention, il est possible de doser chacun des sérotypes présent dans une composition virale, au moyen d'anticorps monoclonaux non neutralisants sans induction d'interférence entre les différents sérotypes.

## Revendications

1. Méthode pour déterminer, dans une composition contenant différentes espèces ou types de virus vivants, la quantité de virus de chacun des types ou espèces de virus, **caractérisée en ce qu'**elle comprend les étapes suivantes :
. propagation des virus de chaque type ou espèce sur des cellules VERO
. dosage de chaque type ou espèce de virus au moyen d'un anticorps monoclonal spécifique.

2. Méthode selon la revendication précédente, **caractérisée en ce que** la composition contenant différentes espèces ou types de virus vivants est une composition vaccinale.

3. Méthode selon une des revendications précédentes, **caractérisée en ce que** la composition contenant différentes espèces ou types de virus vivants est une composition contenant quatre sérotypes de virus vivants atténués de la Dengue.

4. Méthode selon une des revendications 1 à 2, **caractérisée en ce que** la composition contenant différentes espèces ou types de virus vivants est une composition contenant trois sérotypes de virus vivants atténués de la polio.

5. Méthode selon une des revendications 1 à 2, **caractérisée en ce que** la composition contenant différentes espèces ou types de virus vivants est une composition contenant différents types de rotavirus.

## Patentansprüche

1. Methode zur Bestimmung der Virusmenge von jedem Typ oder jeder Art von Virus in einer Zusammensetzung, die verschiedene Typen oder Arten von lebenden Viren enthält, **dadurch gekennzeichnet, daß** sie die folgenden Stufen umfaßt:
- Vermehrung der Viren von jedem Typ oder jeder Art auf Zellen VERO,
- Bestimmung von jedem Typ oder jeder Art von Virus mit Hilfe eines spezifischen monoklonalen Antikörpers.

2. Methode nach dem vorstehenden Anspruch, **dadurch gekennzeichnet, daß** die Zusammensetzung, die verschiedene Typen oder Arten von lebenden Viren enthält, eine vaccinale Zusammensetzung ist.

3. Methode nach einem der vorstehenden Ansprüche, **dadurch gekennzeichnet, daß** die Zusammensetzung, die verschiedene Typen oder Arten von lebenden Viren enthält, eine Zusammensetzung ist, die vier Serotypen von abgeschwächten Lebendviren von Dengue enthält.

4. Methode nach einem der Ansprüche 1 bis 2, **dadurch gekennzeichnet, daß** die Zusammensetzung, die verschiedene Typen oder Arten von lebenden Viren enthält, eine Zusammensetzung ist, die drei Serotypen von abgeschwächten Lebendviren von Polio enthält.

5. Methode nach einem der Ansprüche 1 bis 2, **dadurch gekennzeichnet, daß** die Zusammensetzung, die verschiedene Typen oder Arten von lebenden Viren enthält, eine Zusammensetzung ist, die verschiedene Typen von Rotavirus enthält.

## Claims

1. Method for determining the virus quantity of each of the virus types or virus species in a composition which contains different species or types of live virus, **characterized in that** it comprises the following steps:
• propagating the viruses of each type or species on Vero cells,
• assaying each type or species of virus using a specific monoclonal antibody.

2. Method according to the preceding claim , **characterized in that** the composition comprising different species or types of live virus is a vaccine composition.

3. Method according to either of the preceding claims, **characterized in that** the composition comprising different species or types of live virus is a composition which comprises four serotypes of live attenuated dengue virus.

4. Method according to one of Claims 1 to 2, **characterized in that** the composition comprising different species or types of live virus is a composition which comprises three serotypes of live attenuated polio virus.

5. Method according to one of Claims 1 to 2, **characterized in that** the composition comprising different species or types of live virus is a composition which comprises different types of rotavirus.
